Europäisches Patentamt

European Patent Office

Office européen des brevets

(19)

(11) Veröffentlichungsnummer: **0 049 424**
**B1**

(12) **EUROPÄISCHE PATENTSCHRIFT**

(45) Veröffentlichungstag der Patentschrift:
23.07.86

(51) Int. Cl.⁴: **A 61 K 35/78**, A 61 K 7/00

(21) Anmeldenummer: **81107590.2**

(22) Anmeldetag: **23.09.81**

(54) **Bioaktives, pflanzliche Wirkstoffe enthaltendes Mittel.**

(30) Priorität: **03.10.80 DE 3037526**

(43) Veröffentlichungstag der Anmeldung:
**14.04.82 Patentblatt 82/15**

(45) Bekanntmachung des Hinweises auf die Patenterteilung:
**23.07.86 Patentblatt 86/30**

(84) Benannte Vertragsstaaten:
**AT BE CH DE FR GB IT LI LU NL SE**

(56) Entgegenhaltungen:
**EP-A-0 052 203**
**EP-A-0 054 627**
**DE-A-387 596**
**DE-A-817 335**
**FR-A-931 503**
**FR-A-1 010 633**
**GB-A-459 540**

**HAGERS HANDBUCH DER PHARMAZEUTISCHEN**
**PRAXIS, 4. Auflage, Band 5, "Chemikalien und**
**Drogen (H-M)", 1976, Springer-Verlag, Berlin, DE.**
**The Chemistry and Technology of Waxes - A.H.**
**Warth, Reinhold Publishing Corp. (1947), S.**
**122,123,126-177, 450**
**Römps Chemie-Lexikon, 8. Auflage, Seiten 1305-7**

(73) Patentinhaber: **Füssener Textil AG, Mühlbachgasse
2, D-8958 Füssen/Ostallgäu (DE)**

(72) Erfinder: **Stüber, Siegfried, Dr., Drehergasse 46,
D-8958 Füssen/Ostallgäu (DE)**

(74) Vertreter: **Patentanwälte TER MEER - MÜLLER -
STEINMEISTER, Mauerkircherstrasse 45, D-8000
München 80 (DE)**

## Beschreibung

Die Erfindung betrifft die Verwendung von Flachswachs oder Flachswachsanteilen zur Herstellung eines lokal anzuwendenden Arzneimittels zur Behandlung von Erkrankungen des rheumatischen Formenkreises, des Bewegungsapparates und von Enzündungen sowie von Kosmetika.

Die Rückbesinnung auch der neueren medizinischen Forschung auf die wertvolle Ausnützung natürlicher Kräfte hat dazu geführt, daß Arzneimittel und kosmetische Zubereitungen auf natürlicher, vegetativischer Basis wieder zunehmend an Bedeutung gewonnen haben.

Flachswachs ist in der Literatur schon beschrieben worden (Technologie der Textilfasern, "Der Flachs", Band V, 1,1 (1930) 129 - 141 und 283; Hofmeister, Bir 1903 1047; Ullmanns Enzyklopädie der technischen Chemie, 18. Band (1976) 287). Aus "The Chemistry and Technology of Waxes" A.H. Warth, Reinhold Publishing Corp. (1947) Seiten 122, 123, 366 bis 377 und 450 bis 454 ist es bereits bekannt, daß man Flachswachs mit Vorteil als Ersatz für Bienenwachs in gewissen Rezepturen für Schuhpolituren und Schuhcrems einsetzen kann. Es ist weiterhin in "Hagers Handbuch der Pharmazeutischen Praxis", Band 5, 4. Ausgabe, Springer Verlang (1976), Seiten 517 bis 525 angegeben, daß man aus Leinstroh ein Wachs gewinnen kann, daß in seinen Eigenschaften dem Carnaubawachs ähnelt und das gute Poliereigenschaften aufweist (Seiten 523 und 254).

Die DE-C-817 335 beschreibt ein Verfahren zur Wachsgewinnung aus Flachsabfällen ohne auf die Eigenschaften des Wachses einzugehen. In "Römpps Chemie-Lexikon", 8. Auflage, Seiten 1305 bis 1307 ist schließlich beschrieben, daß Flavone und Flavonoide, daß heißt Pflanzenfarbstoffe, die auch im Flachs vorkommen, zum Teil, wie im Fall von Hesperidin und Naringin, arzneilich gebraucht werden.

Flachswachs besteht im wesentlichen aus Fettsäureestern langkettiger Alkohole und Kohlenwasserstoffen und ist in der Rindenschicht des Flachses zu etwa 10% enthalten. Bei der Flachsaufbereitung, insbesondere beim Knicken und Schwingen des Röststrohs, aber auch beim Grünflachsknicken, bei der Verbaumwollung und auch noch in weiteren Stufen, bis einschließlich dem Verspinnen, fällt ein mehr oder weniger feinteiliges, flachswachshaltiges Material an. Der grobe, Scheben und kurze Wergteile enthaltende Staub enthält Flachswachs in einer Menge bis etwa 10 Gew.-% und der feine und feinste Staub, wie er beispielsweise in der Spinnerei anfällt, in einer Menge bis zu etwa 25 Gew.-%. Es ist möglich, daraus Flachswachs durch Extraktion, beispielsweise in kontinuierlicher Weise mit Lösungsmitteln, wie Aceton, Äthanol, Benzol und anschließendes Abtreiben des Lösungsmittels zu gewinnen.

Überraschenderweise hat sich nun gezeigt, daß Flachswachs bzw. Flachswachsanteile wertvolle pharmakologische und kosmetische Wirkungen entfalten. Insbesondere gilt dies bei der lokalen Anwendung zur Behandlung von Erkrankungen des rheumatischen Formenkreises, wie Ischias, Gicht, Arthriden und Arthrose, Muskelverspannungen, Erkrankungen des Bewegungsapparates, Prellungen, Schwellungen, Entzündungen (Venenscheidenentzündung, Insektenstiche) und dergleichen.

Der vorliegenden Erfindung liegt daher die Erkenntnis zugrunde, daß bei der Flachsaufbereitung anfallende bisher lediglich als lästiges Abfallmaterial betrachtete Produkte wertvolle biologische Wirkungen entfalten.

Gegenstand der Erfindung ist daher die Verwendung von Flachswachs oder Flachswachsanteilen zur Herstellung eines lokal anzuwendenden Arzneimittels zur Behandlung von Erkrankungen des rheumatischen Formenkreises, des Bewegungsapparates und von Entzündungen sowie von Kosmetika.

Bevorzugt bildet man die Arzneimittel oder Kosmetika in Form von Salben, Crems, Packungen, Ölen oder Bädern aus.

Als besonders wirksam haben sich dabei Flachswachsanteile erwiesen, das heißt also Wirkstoffe bzw. Wirkstoffgemische, wie sie beispielsweise im Flachswachs enthalten sind und die erhältlich sind

a) durch Wasserdampfdestillation von flachswachshaltigem Rohmaterial oder von Rohflachswachs, das aus solchem Rohmaterial durch Lösungsmittelextraktion und gegebenenfalls Bildung des lösungsmittelfreien Extrakts gewonnen wurde, insbesondere die bei der Wasserdampfdestillation gewonnene wässrige, spezifisch leichtere und/oder spezifisch schwerere Phase und/oder

b) durch Behandlung von flachswachshaltigem Rohmaterial, bzw. von aus solchem Rohmaterial durch Lösungsextraktion und gegebenenfalls nach Abtreiben des Lösungsmittels gewonnenen Rohflachswachs, gegebenenfalls nachdem diese Stoffe zuvor der Wasserdampfdestillation unterworfen waren, mittels Säuren, Extraktion der dann alkalisch eingestellten wässrigen Lösung mit organischen, polaren Lösungsmitteln, insbesondere 80 - 85°C und Abtreiben des organischen Lösungsmittels und/oder

c) durch fraktionierte Destillation flachswachshaltiger Lösungen in organischen Lösungsmitteln unter Gewinnung einer gegebenenfalls von restlichem Lösungsmittel noch zu befreienden Fraktion von 90-150°C/720 Torr.

Als flachswachshaltiges Rohmaterial kann z.B. das zuvor schon genannte, bei der Flachsaufbereitung, insbesondere beim Knicken und Schwingen des Röststrohs, aber auch beim Grünflachsknicken, bei der Verbaumwollung und selbst noch in weiteren Stufen, wie dem Spinnen, anfallende mehr oder weniger feinteilige Material verwendet werden. Als Rohflachswachs kann das, wie ebenfalls zuvor bereits beschrieben, durch Extraktion gewonnene Flachswachs eingesetzt werden. Bevorzugt werden aber für die Extraktion wegen der besseren Abtrennbarkeit, mit Wasser nicht mischbare, organische, polare Lösungsmittel, insbesondere Halogenkohlenwasserstoffe, wie 1,1,1-Trichloräthan, Methylenchlorid, Chloroform, Tetrachlorkohlenstoff, wenn auch Äther, Äthanol und Methanol sowie Ketone, wie Aceton, nicht ausgeschlossen sind. Ganz allgemein sind mit Wasser nicht mischbare, polare Lösungsmittel mit einem Siedepunkt von kleiner als etwa 80 - 85°C bevorzugt. Ein solches Rohflachswachs kann auch unmittelbar als solches zur Herstellung eines Arzneimittels im Sinne der Erfindung verwendet werdet.

Die Wasserdampfdestillation, insbesondere im Temperaturbereich um oder unter 100°C, z.B. 95 - 100°C, sollte an einem neutralen bzw. schwach basischen Material, was gegebenenfalls durch entsprechende pH-Einstellung erfolgen kann, durchgeführt werden. Bei der Wasserdampfdestillation bildet sich in der Vorlage eine obere (spezifisch leichteste), eine mittlere (wäßrige) und untere (spezifisch schwerste) Phase. Obwohl die mittlere und untere Phase bevorzugt sind, kann im Sinne der Erfindung auch die obere, terpenartig riechende Phase verwendet werden. Die Phasen können unmittelbar als solche oder nach Entfernung des Wassers verwendet werden. Es ist auch möglich, das bei der Wasserdampfdestillation anfallende Destillat einer Extraktion mit Lösungsmitteln, wie den vorgenannten, zu unterwerfen und daraus einen lösungsmittelfreien Flachswachsanteil zu gewinnen.

Für die Säureextraktion von Rohmaterial oder Rohflachswachs empfehlen sich insbesondere verdünnte, wäßrige Mineralsäuren oder wäßrige, niedrige aliphatische Säuren, wie 0,1 - 1 n Salzsäure oder wäßrige Essigsäure. Für die Extraktion der durch Säurebehandlung, vorzugsweise von unlöslichen Bestandteilen befreiten und dann alkalisch, insbesondere pH 7,5 - 11, gemachten Lösung werden die gleichen Lösungsmittel bevorzugt wie zur Herstellung des Rohflachswachses, also mit Wasser nicht mischbare organische Lösungsmittel, wie Halogenkohlenwasserstoffe, Alkohole, Ketone usw., namentlich eines Siedepunkts von kleiner als ungefähr 80 - 85°C.

Als Flachswachsanteile können erfindungsgemäß auch Flachsöle, venvendet werden wie sie beispielsweise mittels fraktionierter Destillation flachswachshaltiger, organischer Lösungen durch Gewinnung der Fraktion von 90 - 150°/720 Torr erhältlich sind. Die zur Herstellung der Lösungen verwendeten organischen Lösungsmittel weisen einen Siedepunkt außerhalb des Bereichs von 90 - 150°/720 Torr auf und können wiederum die vorgenannten organischen Lösungsmittel sein oder solche mit einem Siedepunkt von ober halb 150°C/720 Torr wie z.B. Dibutylketon.

Die durch Wasserdampfdestillation, saure Extraktion oder fraktionierte Destillation erhaltenen Produkte können noch in üblicher Weise weiter gereinigt werden, z.B. durch Absorptionsverfahren, Feindestillation usw.

Die aus Flachswachs gewinnbaren Anteile bzw. Stoffe enthalten offensichtlich bioaktiv besonders wirkungsvolle Komponenten, wobei nicht völlig ausgeschlossen werden kann, daß sich bioaktive Wirkkomponenten, möglicherweise zum Teil, während der Behandlung erst noch bilden. Regelmäßig weisen die durch die vorgenannten Verfahren der Wasserdampfdestillation (nach Entwässerung), Säureextraktion oder fraktionierten Destillation gewonnenen Produkte ölige Konsistenz (Flachsöle) auf.

In den so behandelten Flachswachsrückständen befinden sich jedoch immer noch spürbare Mengen an Flachsölen. Gegenstand der Erfindung ist demgemäß auch verwendung solcher Flachswachsanteile, die gegenüber den Ausgangsflachswachsen hinsichtlich des Flachsölgehalts erniedrigt oder davon freie Flachswachse enthalten, insbesondere soweit die Verringerung des Flachsölanteils durch Wasserdampfdestillation, Säureextraktion oder fraktionierte Destillation, gemäß der vorbeschriebenen Verfahrensweise a), b) und/oder c) erfolgte. Wird hierbei von Rohflachswachs ausgegangen, so fallen die an Flachsöl verarmten bzw. befreiten Flachswachsanteile als solche an. Wird von flachswachshaltigem Rohmaterial ausgegangen (Wasserdampfdestillation; Säureextraktion) so können die so behandelten Rückstände mit den hierfür bekannten Lösungsmitteln extrahiert werden unter Gewinnung des lösungsmittelbefreiten Flachswachsanteil-Extrakts in üblicher Weise.

Die erfindungsgemäßen vervendeter Flachswachse bzw. Flachswachsanteile können mit bekannten Excipientien, Hilfsstoffen, Trägern, Konservierungsmitteln und dergl. in üblicher Weise konfektioniert werden. Dabei ist es beispielsweise zur Bereitung von Bädern durchaus möglich, Flachswachs enthaltenden Staub unmittelbar dem Bad zuzusetzen oder in einen Filterbeutel einzubringen. Heilsalben haben sich wegen des coenzymartigen Charakters der im Flachswachs, insbesondere in den Flachswachsanteilen enthaltenen Wirkstoffe in besonderer Weise bewährt, wobei die Verwendung von im Flachswachs enthaltenen, mit den Vitaminen verwandten Stoffen als Salbengrundlage, auch in kosmetischen Salben (Beseitigung von Ekzemen, Hautstraffung), wesentlich zur bioaktiven Wirkung beiträgt. Bemerkenswert in diesem Zusammenhang ist auch das hohe Wasserbindevermögen bzw. die hohe Emulgierkraft des erfindungsgemäßen verwendeten Flachswachses bzw. Flachswachsanteils. Außerdem weist die Salbe ein hohes Resorptionsvermögen in der Haut auf.

Die Gewinnung von Flachswachsanteilen kann beispielsweise in folgender Weise erfolgen.

a) 100 g Flachsstaub (Nasser Flachsstaub, pH

8) werden 3 Stunden der Wasserdampfdestillation unterworfen. Dabei konnte aus 1,5 Liter Destillat, etwa 2 g Extrakt mittels mehrmaliger Extraktion (5-mal) mit Äther und Abziehen des Äthers am Rotationsverdampfer gewonnen werden.

b) 100 g Flachsstaub werden mit 500 ml 0,6 n HCl versetzt, 1/2 Stunde stehengelassen und abfiltriert. Es wird noch zweimal mit je 200 ml heißem (80°C) Wasser versetzt und abfiltriert. Die Lösung wird mit 0,6 n NaOH alkalisch gemacht (pH 9) und mit Methylenchlorid oder Äther extrahiert (5-mal). Nach Abziehen des Lösungsmittels am Rotationsverdampfer werden etwa 4 g öliges Produkt erhalten.

c) Zur kontinuierlichen Extraktion werden 100 g Flachsstaub mit 400 ml Lösungsmittel (1,1,1-Trichloräthan) behandelt und die Entfernung des größten Teils des Lösungsmittels erfolgt am Rotationsverdampfer (Wasserbadtemperatur 25°C). Beim Ausfallen des Flachswachses wird die Badtemperatur auf 85°C gesteigert und es geht öliges produkt mit etwas Lösungsmittel über. Die Destillation wird bis zum Schmelzen des Wachses fortgesetzt. Das restliche Lösungsmittel in der Vorlage wird am Rotationsverdampfer bei einer Badtemperatur von 25°C abgezogen. Es werden 2,5 g eines öligen Produkts erhalten.

Ein Teil des Flachsölgemisches ($Kp_{720}$: 90 - 150°C) bleibt nach obigen Behandlungen im Flachswachs zurück. Wurde Flachswachs gewonnen, mit oder ohne Austreiben von Flachsölen, so empfiehlt es sich, insbesondere zur Salbenverarbeitung das Wachs nochmals z.B. mit Alkoholen wie Äthanol zu lösen und am Rotationsverdampfer abzuziehen.

Die nachfolgenden Beispiele dienen der Erläuterung der Erfindung.

## 1. Salbe

Unter leichtem Erwärmen werden die nachfolgenden Bestandteile gemischt
Flachswachs natur, grün 15 %
Glycerin 5 %
Neutralöl (Triglyceride; 75 %
Sonnenblumenöl)
Äthanol 3 %
wäßrige Lösung von Vitamin C
Konzentration wie im Zitronensaft (oder Zitronensaft selbst) 0,5 %
Eiweiß (Kollagen oder
Hühnereiweiß) 1,5 %
Für kosmetische Zubereitungen kann auch Wasser, z.B. 5 - 10 %, in die Salbe eingearbeitet werden (Feuchtigkeitscreme zur Straffung der Haut).

Die Salbe hat sich besonders bewährt bei Gelenkschmerzen, Gicht, Rheuma, Arthrose, Muskelverspannungen und Bandscheibenbeschwerden. Zudem ist die Salbe durch die in ihr enthaltenen Flachsflavyliumfarbstoffe, die zur Gruppe der Phenole gehören und deshalb konservierend wirken, also durch natürliche Konservierungsmittel, vor bakteriellem und mycotischem Abbau geschützt. Die Farbe der Salbe ist je nach eingesetztem Flachswachs variabel, der Geruch nach Flachs kann entzogen werden. Dies kann beispielsweise durch Vakuumbehandlung in Gegenwart von geruchsbindenden Stoffen, durch Umkristallisation in Gegenwart von Absorptionsmitteln erfolgen.

Zur Herstellung von Feuchtigkeitscremes kann Flachswachs in ein nicht fettendes, als Salbengrundlage dienendes, Gel eingearbeitet werden.

## 2. Öl oder Balsam.

Das durch die angegebene Behandlung erhältliche, bevorzugte Flachsöl kann ganz einfach mit Neutralöl (Sonnenblumenöl, Olivenöl etc.) verdünnt werden (Verhältnis Flachsöl: Sonnenblumenöl 1: 15 bis 1: 20). Die Zubereitung hat sich insbesondere für Massagezwecke bei rheumatischen Erkrankungen sehr bewährt.

## 3. Rheumabad.

Flachswachshaltiger Staub wird nach Einstellung eines schwachsauren pHs mittels eines Acetatpuffers in einem Filterbeutel verpackt. Beim Einbringen des Filterbeutels in der Badewanne werden Wirkstoffe sofort abgegeben.

## 4. Packungen.

Flachswachshaltiger Staub wird mit einem Acetatpuffer schwachsauer eingestellt und die Masse in einen Beutel eingebracht. Nach Anfeuchten mit Wasser und Erhitzen sind Packungen erhältlich, die insbesondere bei Bandscheibenbeschwerden gute Wirkung gezeigt haben.

## 5. Haarwasser

Die bei der Wasserdampfdestillation oder den anderen Methoden erhältlichen wäßrigen und organischen Phasen werden durch Verschneiden mit Alkoholen, wie Isopropanol, in Haarwasser überführt, welches den Stoffwechsel der Haarwurzeln anregt.

**Patentansprüche**

1. Verwendung von Flachswachs oder Flachswachsanteilen zur Herstellung eines lokal anzuwendenden Arzneimittels zur Behandlung von Erkrankungen des rheumatischen Formenkreises, des Bewegungsapparates und von Entzündungen sowie von Kosmetika.

2. Verwendung nach Anspruch 1 des Arzneimittels oder der Kosmetika in Form von Salben, Cremes, Packungen, Ölen oder Bädern.

**Claims**

1. The use of flax wax or flax wax components for the manufacture of a pharmaceutical composition to be locally applied for the treatment of diseases of rheumatic nature, of the apparatus of locomotion and of inflammations and of cosmetics.

2. The use according to claim 1 of the pharmaceutical composition or the cosmetics in the form of ointments, cremes, packages, oils or baths.

**Revendications**

1. Utilisation de cire de lin ou de fractions de cire de lin pour la fabrication d'un médicament applicable localement pour le traitement de maladies de type rhumatismal, de l'appareil locomoteur et d'inflammations, ainsi que de produits cosmétiques.

2. Utilisation selon la revendication 1 du médicament ou du produit cosmétique sous forme de pommades, de crèmes, d'enveloppements, d'huiles ou de bains.